# EUROPEAN PATENT APPLICATION

(11) **EP 1 669 075 A1**
(43) Date of publication of application: **14.06.2006**
(21) Application number: 04722723.6
(22) Date of filing: 23.03.2004
(51) Int. Cl.: A61K 31/57, A61K 9/06, A61K 9/08, A61P 27/00, C07J 7/00

(54) **THERAPEUTIC AGENT FOR AGEING MACULAR DEGENERATION**

(30) Priority: 29.09.2003 JP 2003337426
(71) Applicant: Meiji Dairies Corporation, Tokyo 136-8908 (JP)
(72) Inventor: HIBINO, Satoshi, Fukuyama-shi, Hiroshima 7290292 (JP); YAMADA, Masashi, Koto-ku, Tokyo 1368908 (JP); YAMAJI, Taketo, Odawara-shi, Kanagawa 2500862 (JP); SUZUKI, Hirohito, Koto-ku, Tokyo 1368908 (JP)
(74) Representative: Hartz, Nikolai
(86) International application number: PCT/JP2004/003969
(87) International publication number: WO 2005/030221

(57) **Abstract**

Provided is a medicament having an excellent therapeutic effect on ageing macular degeneration. The therapeutic agent for ageing macular degeneration comprises a progesterone derivative represented by the following formula (1): 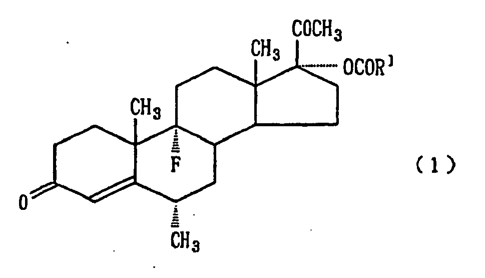 (wherein, R¹ represents an alkyl group having from 1 to 23 carbon atoms) as an effective ingredient.

## Description

### Technical Field

The present invention relates to a therapeutic agent for ageing macular degeneration.

### Background Art ,

Ageing macular degeneration occurs mostly after age 50. It is an intractable and unexplained eye disease which ranks as the number-one cause for vision loss in Western people who are more sensitive to light stimulation than the Japanese. The number of the patients suffering from it is said to 10 million or more. The number of the onset of this disease is increasing also in Japan owing to the westernization of lifestyle, an increase in the opportunities to receive an optical stimulus with the diffusion of TV or personal computers, increase in average life expectancy and the like. In this ageing macular degeneration, abnormal ageing of the retinal pigment epithelial cells deteriorates the macular region, which causes symptoms such as fuzzy or distorted view at the central portion of the visual field, loss of the central view, and darkening of the view.

The ageing macular degeneration has two forms, that is, atrophic and exudative forms. In the former one, the macular region is damaged and therefore atrophies. No particular treatment is necessary because the patient have no subjective symptoms of the disease. In the exudative form, on the other hand, new abnormal blood vessels grow by the stimulation of waste products and the retina changes its shape. As a result, retinal pigment epithelium detachment, choroidal neovascularization and subretinal hemorrhages leading to visual loss occur so that it needs proper treatment. Treatments of it include surgical therapies such as laser photocoagulation, removal of abnormal blood vessels from the choroid and translocation of the macular region, and temporary therapies such as low-intensity radiation therapy, photodynamic therapy and transpupillary thermo-therapy. Treatments by a medicament, on the other hand, include administration of a hemostat to prevent hemorrhage and administration of a vitamin preparation to supply nourishment to the retina. These treatments are however only symptomatic therapies and are not definitive ones.

### Disclosure of the Invention

An object of the present invention is to provide a medicament directly acting on ageing macular degeneration.

The present inventors have searched for a medicament effective for the treatment of ageing macular degeneration. As a result, it has been found that a progesterone derivative represented by the below-described formula (1) has an excellent inhibitory action on choroidal neovascularization without adversely affecting a retinal function and is highly effective for amelioration or treatment of ageing macular degeneration symptoms, leading to the completion of the present invention.

In the present invention, there is thus provided a therapeutic agent for ageing macular degeneration, which comprises as an effective ingredient a progesterone derivative represented by the following formula (1): 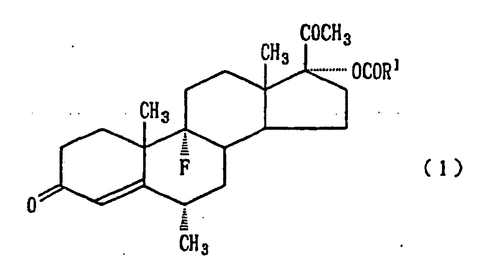 (wherein, R¹ represents a hydrocarbon group having from 1 to 23 carbon atoms).

In the present invention, there is also provided the use of a progesterone derivative represented by the above-described formula (1) for the preparation of a therapeutic agent for ageing macular degeneration.

In the present invention, there is also provided a treating method of ageing macular degeneration, which comprises administering an effective amount of a progesterone derivative represented by the above-described formula (1).

The therapeutic agent for ageing macular degeneration according to the present invention has an excellent inhibitory effect on choroidal neovascularization, is highly effective for ameliorating or treating ageing macular degeneration symptoms such as appearance of fuzzy or distorted areas in the central vision owing to degeneration of the macular region of the retina. It is particularly useful as a therapeutic agent for the exudative ageing macular degeneration.

### Brief Description of the Drawings

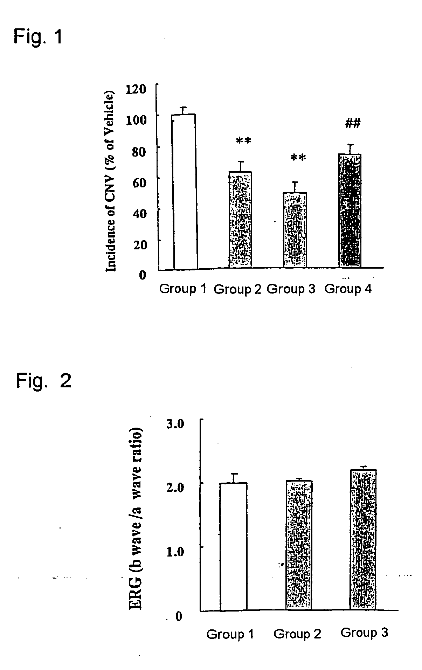

FIG. 1 illustrates an inhibitory effect on CNV development of rats brought about by the administration of FMPA; and FIG. 2 illustrates a change in an ERG b-wave/ERG a-wave amplitude ratio brought about by the administration of FMPA.

### Best Mode for Carrying out the Invention

The progesterone derivative represented by the formula (1) is described in WO95/26974. It is known to have an inhibitory action on neovascularization and is useful as a therapeutic agent for malignant tumor, diabetic retinopathy, rheumatism and the like. It is however not known that it has a therapeutic effect for ageing macular degeneration.

Examples of the hydrocarbon group R¹ in the formula (1) include linear, branched or cyclic alkyl or alkenyl groups having from 1 to 23 carbon atoms. Of these, alkyl groups having from 1 to 17 carbon atoms are preferred, of which alkyl groups having from 1 to 7 carbon atoms such as methyl, ethyl, n-propyl, isopropyl, n-butyl, n-pentyl and n-heptyl are more preferred as R¹. Especially, 9α-fluoromedroxyprogesterone acetate (which may hereinafter be called FMPA) having a methyl group as R¹ is preferred.

The progesterone derivative represented by the formula (1) is prepared in accordance with the process as described in WO95/26974.

The progesterone derivative represented by the formula (1) can, as shown later by Examples, strongly suppress choroidal neovascularization which is thought to be a cause of ageing macular degeneration and suppress degeneration of the macular region of the retina so that it is useful as a therapeutic agent for ageing macular degeneration, especially, exudative ageing macular degeneration.

The therapeutic agent for ageing macular degeneration according to the present invention is prepared in a manner known *per se* in the art by mixing the progesterone derivative represented by the formula (1) serving as an effective ingredient with an ordinarily employed pharmaceutical vehicle.

Examples of the pharmaceutical vehicle include aqueous or non-aqueous solvent, solubilizing agent, stabilizer, preservative, surfactant, soothing agent, buffer, suspending agent and thickener.

The therapeutic agent for ageing macular degeneration according to the present invention is provided preferably as a liquid or ointment.

The therapeutic agent for ageing macular degeneration according to the present invention is provided preferably in the ophthalmologically acceptable form such as eye drop, injection solution to Tenon's capsule and injection solution to ocular fundus.

When the therapeutic agent is provided as an ophthalmic solution, another pharmaceutically acceptable component may be added as needed insofar as it does not cause eye pain. Examples include anti-inflammatory agents such as epsilon-aminocaproic acid, dipotassium glycyrrhizinate, dicrofenac sodium and pranoprofen; vasoconstrictors such as phenylephrine hydrochloride, naphazoline hydrochloride and tetrahydrozoline hydrochloride; anti-allergic drugs such as sodium cromoglicate and ketotifen fumarate; antihistamine drugs such as chlorpheniramine maleate and diphenhydramine hydrochloride, antiseptics such as benzalkonium chloride, paraoxybenzoate ester, sorbic acid and chlorobutanol; surfactants such as polyoxyethylene hydrogenated castor oil and polyoxyethylene sorbitan monooleate; vitamins such as pyridoxine hydrochloride, riboflavin phosphate, cyanocobalamin, panthenol, tocophenol acetate, and flavin adenine dinucleotide sodium; amino acids such as sodium chondroitin sulfate, potassium L-aspartate, and aminoethylsulfonic acid; and inorganic salts such as sodium chloride and potassium chloride.

The pH of the agent may be adjusted as needed with a pH regulator such as hydrochloric acid, phosphoric acid, citric acid, sodium hydroxide or sodium bicarbonate.

It is preferred from the standpoint of the therapeutic effect for ageing macular degeneration that the therapeutic agent for ageing macular degeneration according to the present invention is administered at a dose of from 0.01 to 300 mg, in terms of the compound of the formula (1), in one to five portions, though depending on the symptoms, age, weight or the like of the patient.

### Examples

The present invention will hereinafter be described in detail by Examples. It should however be borne in mind that the present invention is not limited to or by these Examples.

### Example 1

An inhibitory effect on choroidal neovascularization caused by the subconjunctival administration of FMPA was assessed using a rat model of laser-induced choroidal neovascularization (which may hereinafter be abbreviated as CNV).

### (1) Production of CNV rat model

Rats (Brown Norway, 8 week old, male) were given general anesthesia and their pupils were dilated with one drop of commercially available eye-drops ("Mydrin P", trade name; product of Santen Pharmaceutical Co. Ltd.). They were then subjected to photocoagulation by a krypton laser photocoagulation apparatus ("MC-7000L", manufactured by NIDEK Co., Ltd.). The photocoagulation was performed sporadically at 8 sites while focusing on the deep layer of the retina and avoiding laser irradiation to thick retinal blood vessels (coagulation conditions: spot size of 100 µm, output of 100 mW and coagulation time for 0.1 second). This photocoagulation was given to both eyes. After photocoagulation, the sites exposed to laser were confirmed by fundus photography. These rats were bred for 14 days, whereby CNV rat models were produced.

### (2) Administration of test medicament

After photocoagulation, 50 µL/eye of a solution obtained by dissolving a test medicament in a base was administered once to the upper conjunctivitis of the eye balls through a microsyringe equipped with a 30-gauge needle. Each test medicament was administered to four rats.

The base employed here was an aqueous solution containing 0.4 wt.% Tween 80 and 2.6 wt.% concentrated glycerin.

The test medicament was administered to four groups, that is, a base group (Group 1), a group (Group 2) administered with 1000 µg/eye of FMPA, a group (Group 3) administered with 3000 µg/eye of FMPA, and a group (Group 4) administered with 1000 µg of a comparative medicament ("AL3789", product of Alcon, Inc.).

The "AL3789" employed as the comparative medicament is a steroid compound having the following structural formula:

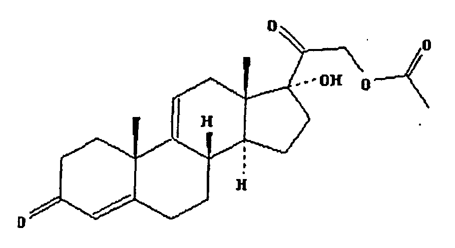

### (3) Assessment method

Fourteen days after photocoagulation, 0.1 mL of 10 wt.% fluorescein was injected through the caudal vein and based on the photograph taken by a fluorescein fundus camera ("Pro III", product of Kowa Company Ltd), presence or absence of CNV was observed. The rats having clouded eyeballs were omitted from the judgment of CNV. When the fluorescein leakage from the exposed site was not observed, the eyeball was judged to be negative for CNV. When the fluorescein leakage was observed, the eyeball was judged to be positive for CNV. A ratio of the number of positive sites to the number of all the exposed sites (8 sites) of eyeballs was calculated and the incidence of CNV of each administration group was calculated when the CNV incidence of the base group (Group 1) was assumed to be 100. It was designated as the incidence (%) of CNV. When the exposed site showed mild hyperfluorescence, two such sites were viewed as one positive site.

### (4) Statistical analysis

The incidence of CNV was expressed as a mean value ± S.E. Comparison between the base group (Group 1) and each FMPA group (Groups 2 and 3) was made using analysis of variance, followed by Dunnett multiple comparison tests, while comparison between the base group and Comparative medicament group (Group 4) or comparison between the FMPA group (Group 2) and Comparative medicament group was made using t-test. The significant level was set at 5% (two-tailed).

The results are shown in FIG. 1.

The administration group of FMPA which is a therapeutic agent for ageing macular degeneration according to the present invention exhibits a dose-dependent CNV inhibitory effect. In the 1000 µg/eye administration group (Group 2) and 3000 µg/eye administration group (Group 3), the incidences of CNV are 62.2 ± 6.7% and 48.9 ± 6.5% relative to that of the base group, respectively. Each group thus exhibits a statistically significant (P<0.01 significant) CNV inhibitory effect.

### Example 2

After completion of the assessment in Example 1, an influence of a therapeutic agent for ageing macular degeneration on the retinal function was measured by electroretinogram (ERG).

### Measuring method

The rats after the completion of the measurement in Example 1 were bred for at least one hour in a dark room and adapted to darkness. The below-described operation was performed under a red light in the dark room. The rats were given general anesthesia and their pupils were dilated by administering an eye drop as in Example 1. After one drop of a 0.4 wt.% solution of Benoxil (trade mark; product of Santen Pharmaceutical Co. Ltd.) was administered to the rats under anesthesia, they were fixed to a measuring stand. A ground electrode (needle electrode) was placed on their tail, while an indifferent electrode (needle electrode) was placed on their nose. An LED electrode was attached to the eyes of the rat and then, the red light was turned off. After confirmation of the recording condition on an oscilloscope, ERG was recorded (optical stimulation time: 150 mmsec, luminescent brightness: 3000 cd/m² (500 µW)).

By using an ERG measuring apparatus ("NEC SYNAX ER1100", product of NEC), the amplitude of each of a-wave and b-wave was measured based on the waveform thus obtained. A ratio (b/a ratio) of the ERG b-wave amplitude to the ERG a-wave amplitude was calculated, followed by the statistical analysis as in Example 1.

The results are shown in FIG. 2.

No change in the b/a ratio was observed even by the administration of the therapeutic agent for ageing macular degeneration according to the present invention, suggesting that the administration had no adverse effect on the retinal function.

### Example 3

An eye drop having the following composition was prepared:

| | | |
|---|---|---|
| Composition: | FMPA | 0.05 wt.% |
| | Sodium chloride | 0.08 |
| | Sodium dihydrogen phosphate | 0.001 |
| | Benzalkonium chloride | 0.0001 |
| | Purified water | Balance to 100 |

## Claims

1. A therapeutic agent for ageing macular degeneration, which comprises as an effective ingredient a progesterone derivative represented by the following formula (1): 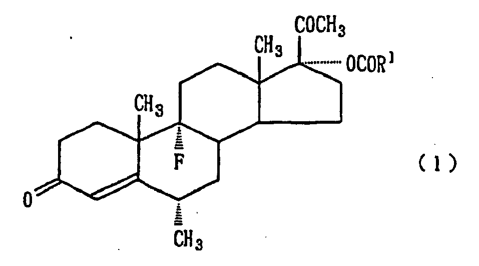 (wherein, R¹ represents an alkyl group having from 1 to 23 carbon atoms).

2. A therapeutic agent for ageing macular degeneration according to Claim 1, wherein the progesterone derivative is 9*α*-fluoromedroxyprogesterone acetate.

3. A therapeutic agent for ageing macular degeneration according to Claim 1 or 2, wherein the ageing macular degeneration is provided as an eye drop, injection solution to Tenon's capsule, injection solution to ocular fundus, or ointment.

4. Use of a progesterone derivative represented by the following formula (1): 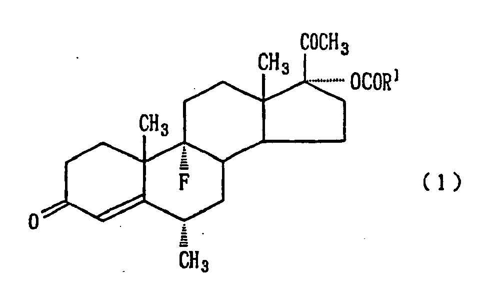 (wherein, R¹ represents an alkyl group having from 1 to 23 carbon atoms) for the preparation of a therapeutic agent for ageing macular degeneration.

5. Use according to Claim 4, wherein the progesterone derivative is 9α-fluoromedroxyprogesterone acetate.

6. Use according to Claim 4 or 5, wherein the ageing macular degeneration is provided as an eye drop, injection solution to Tenon's capsule, injection solution to ocular fundus, or ointment.

7. A treating method of ageing macular degeneration, which comprises administering an effective amount of progesterone derivative represented by the above-described formula (1): 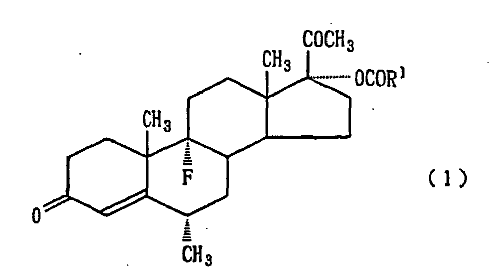 (wherein, R¹ represents an alkyl group having from 1 to 23 carbon atoms).

8. A treating method according to Claim 7, wherein the progesterone derivative is 9*α-*fluoromedroxyprogesterone acetate.

9. A treating method according to Claim 7 or 8, wherein administration is done in the form of an eye drop, injection solution to Tenon's capsule, injection solution to ocular fundus, or ointment.
